# EUROPEAN PATENT APPLICATION

(11) **EP 3 753 486 A2**
(43) Date of publication of application: **23.12.2020**
(21) Application number: 20176914.8
(22) Date of filing: 27.05.2020
(51) Int. Cl.: A61B 5/04, A61B 5/0478, A61B 5/00

(54) **DRY ELECTRODE AND DEVICE FOR MEASURING BIOELECTRICAL SIGNALS**

(30) Priority: 28.05.2019 IT 201900007407
(71) Applicant: Camapanale, Vincenzo, 10024 Moncalieri (TO) (IT); Riente, Fabrizio, 11029 Verrès (AO) (IT); Garlando, Umberto, 15048 Valenza (AL) (IT)
(72) Inventor: Camapanale, Vincenzo, 10024 Moncalieri (TO) (IT); Riente, Fabrizio, 11029 Verrès (AO) (IT); Garlando, Umberto, 15048 Valenza (AL) (IT)
(74) Representative: Carloni, Franco

(57) **Abstract**

A dry electrode (108) for measuring bioelectrical signals comprises an electrode body (200) and a contact element (203) intended to be placed into contact with a portion of the scalp of a user. The contact element (203) is connected in an articulated and sliding manner with respect to said electrode body (200) and is urged by elastic means (206) interposed between said electrode body (200) and said contact element (203), for adjusting the inclination of said contact element (203) with respect to a portion of a user's scalp. The electrode body (200) includes an electronic circuit (209) for amplifying the electrical signal detected by said contact element (203) of the electrode (108) and for decoupling the impedance. The elastic means (206) interposed between said electrode body (200) and said contact element (203) consist of, or include, an electrically conductive material. The electrical signal detected by said contact element (203) is transmitted to the electronic circuit (209) by said elastic means (206). For the articulated connection of said contact element (203) to said electrode body (200) there are provided a conical through-hole in the contact element (203) and an elongated element (204) inserted in said conical through-hole in the contact element, wherein said elongated element (204) is fixed at one end to said electrode body (200) and has at its opposite end an enlargement which engages with said conical hole of the contact element (203) in such a way to allow said contact element (203) to rotate and slide relative to the axis of the elongated element.

## Description

### Technical Field

The present invention refers to a dry electrode for measuring EEG bioelectric signals, and to a device incorporating such electrode.

### Background Art

Conventional electrodes used for measuring bioelectric signals, such as signals recorded in an electroencephalogram (EEG), can be classified into passive and active electrodes.

A passive electrode consists of an electrically conductive terminal part that detects the electrical signal. This signal is transmitted via an electrical connection to a remote electronic circuit board that processes the signal. This solution allows making electrodes at low cost, but these are more susceptible to electrical noise. Accordingly, this requires the use of complex numerical algorithms for eliminating the noise, but this can be eliminated only partially. In order to reduce the impedance between the electrode and the scalp, a passive electrode requires the use of a saline solution or a conductive gel at the interface between the electrode and the scalp. Over time, the gel or saline solution dries out, requiring a new application. This operation is very laborious, requires the intervention of an assistant and a significant amount of time. Although it may be acceptable in a clinical setting, it is impractical for daily use.

An active electrode differs from a passive electrode in that it incorporates an amplification circuit inside the electrode. This allows the signal to be carried at much lower impedance and, therefore, makes it much less susceptible to noise. An active electrode can be either resistive or capacitive. An active resistive electrode requires direct contact between the scalp and the electrode for signal transfer. A capacitive electrode, on the other hand, does not come into direct contact with the patient's scalp, but uses a capacitive connection between the scalp and the electrode.

As already mentioned, the main characteristic of an active electrode is that it exploits the impedance transformation principle at the electrode level. The contact point of the electrode can be connected to an operational amplifier with high input impedance. The high impedance of the operational amplifier allows avoiding that the amplitude and the signal-to-noise ratio of the acquired signal are reduced by the impedance at the interface between scalp and electrode significantly. An active electrode, therefore, does not require the use of a saline solution or gel to lower the contact impedance between the electrode and scalp.

The resistive active electrode usually has a contact element that must reach the patient's scalp. The different shape of the patient's skull may create problems of contact with the electrode or cause an inconvenience to the patient. Furthermore, the presence of hair may interfere with the electrical contact and, therefore, reduce the quality of the signal acquired.

A solution typically adopted for keeping the electrode in contact with the scalp is the use of an elastic cap inside which the electrodes are applied. The elastic cap imparts the force necessary to maintain good contact between the scalp and the electrode surface.

Some known traditional solutions involve the use of an array of telescopic tips positioned at the end of the electrode. As they come into contact with the scalp, they compress and adapt to the morphology of the patient's head.

In particular, WO2016/166740A1 discloses an electrode consisting of a matrix of 32 retractable telescopic tips, each of which is compressed when subjected to pressure. To provide mobility along the longitudinal and transverse axis of the electrode, the electrode is positioned inside an elastic cap. The rotational movement of the electrode is provided solely by the elasticity of the cap. A conductive wire is used to connect the electrode to an electronic circuit board that performs the acquisition. The electrode is of the dry type, but does not have an active electronic circuit integrated in the electrode itself.

US2014/0288406A1 proposes various flexible geometric comb structures (L, circular, etc.) to facilitate the penetration of a metal electrode into the patient's hair. In order to keep the electrodes in contact with the scalp, an elastic cap is used, inside which the electrodes are press-fitted. They are connected by means of a conductor wire to an electronic circuit board which acquires the signals.

In US2014/051044A1 different solutions are proposed for the acquisition of EEG signals through the use of a device composed of two or more adjustable bands, each of which can contain a plurality of electrodes. However, none of the proposed solutions mentions an active electronic circuit integrated inside the electrode (see in particular Fig. 29 of the above mentioned document).

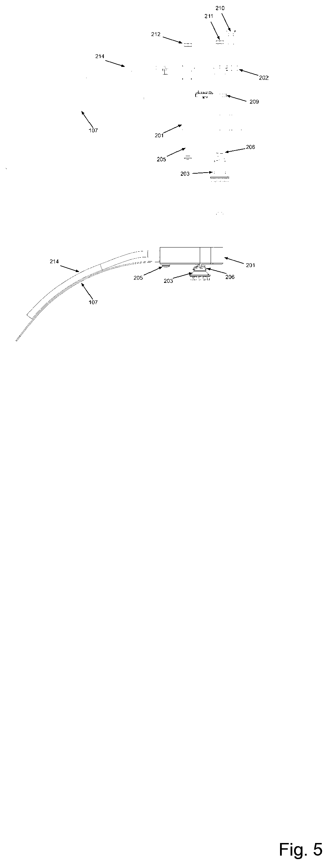

In WO2008/109694A1, from paragraph [0067] to paragraph [0088], different solutions are proposed that allow realizing an electrode for the acquisition of EEG signals. In particular, the electrode presented in Figures 5A-F shows some similarities with the present invention. In this solution, the electrode includes a printed circuit board (PCB) and a flexible conductive element (spring) that electrically connects the mobile part in contact with the scalp and the PCB. However, in this solution, the flexible element is enclosed and held in compression by the contact element at its end, which is locked inside a holder. Even if a spring is present, the friction exerted by the support against the contact element limits the movement thereof. Furthermore, in the above mentioned invention, the end of the electrode consists of a hydro gel. Another limitation of the above mentioned invention is the way in which the contact part of the electrode is bound to the support. In particular, if sufficient torsion force is applied, the contact element could accidentally come out of the holder or get stuck inside in a configuration unsuitable for use.

US2009/0105576A1 proposes the same solutions for the electrode already presented in WO2008/109694A1.

WO2016/200871A1 describes a system consisting of a headset and several sensors. The proposed headset is suitable for use with both children and adults, and is designed for recreational activities that can, for example, train the attention of a subject.

Finally, in US2009/0112077A1 there is described a solution similar to that of document WO2008/109694A1, but with the use of a conical spring.

### Summary of invention

In an EEG signal acquisition system it is important to maintain electrical contact between the electrodes and the subject's scalp. By the term "electrode" we mean a device that allows the acquisition of bioelectric signals from a subject.

The present invention proposes a dry electrode composed by a part in contact with the scalp of the subject and by an electronic circuit, placed inside the electrode itself, which takes care of decoupling the impedance of the acquired signal. In order to improve contact with the subject's irregular cranial surface, in this invention means are provided for ensuring that the electrode is suitably positioned and for giving the contact part two additional degrees of rotational freedom with respect to the body of the electrode itself, which includes the electronic circuit and the housing.

Normally, in order to ensure adhesion, the electrode is anchored to a support that imparts a force along the longitudinal axis of the electrode. In clinical environments, caps with specific anchoring means that hold the electrodes in place are widely used. Such caps can accommodate a large number of electrodes but are not suitable for non-clinical use, where speed of application and ease of use are required. For this reason it is proposed to use lamellae a support for the electrodes. These lamellae are used to connect a multitude of electrodes to two supports placed above the subject's ears. The lamellae are preformed in such a way that, once deformed with respect to their initial configuration, they apply force to the subject's head. The lamellae can be made of preformed spring steel or other material, even non-metallic, with similar elasticity characteristics.

The degree of freedom provided by the deformation of a lamella is not sufficient to ensure uniform electrode contact if the user's head surface is not perfectly perpendicular to the longitudinal axis of the electrode. To further improve the quality of the contact and the comfort of the subject, the following solution is proposed.

An elastic conductive component (compression spring) is positioned between the electrode contact element and the electronic board. In this way, the contact element can rotate with respect to its transverse axes ensuring that the contact element is perpendicular to user's head surface. The contact element can be fixed to the electrode housing by means of a screw . The screw is not responsible for the conduction of the signal but serves to prevent the contact element from moving away due to the thrust of the compression spring.

### Brief description of drawings

The invention will now be described in more detail with reference to the attached drawings:
- Figure 1 shows a headset for reading EEG signals and its main parts,
- Figure 2 shows a front and side elevation view of an electrode according to the present invention for the acquisition of a bioelectric signal,
- Figures 3 shows a bottom and perspective view of the electrode in Figure 2,
- Figure 4 is a sectional view of the electrode in Figure 2, and
- Figure 5 shows an exploded view and a side view of the electrode in Figure 2 and its preformed lamella designed to press the electrode onto a user's head surface, respectively.

### Description of embodiments

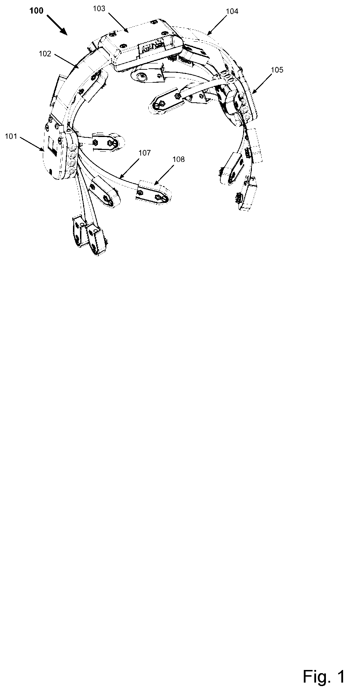

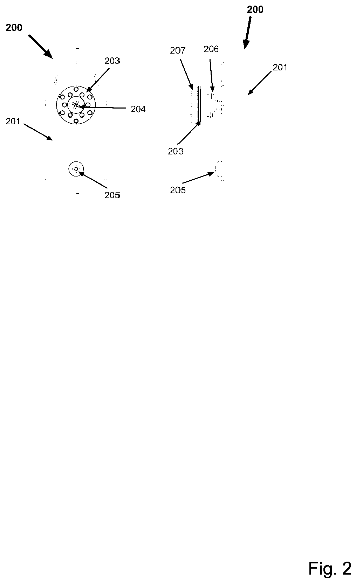

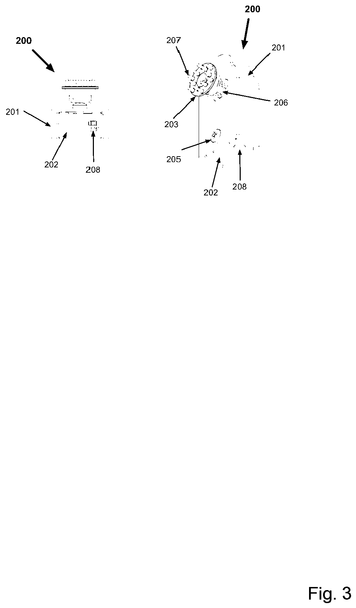

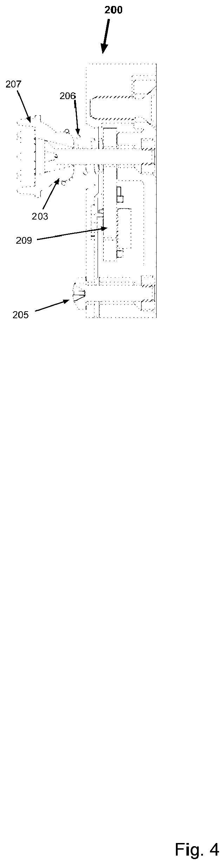

Figure 1 shows an example of an EEG signal reading device, shown as a whole with 100. This device essentially consists of an arc-shaped element that, resting on the head of a subject under examination, holds the electrodes 108 close to the user's head. The device 100 has a central body 103 which contains the main electronic circuit board. The main electronic circuit board processes the digital EEG signals and transmits them, via a wireless communication protocol, to a remote computer. From the central body 103 extend two flexible arc-shaped parts 102 and 104 which can be made of plastic or other suitable material. The two arc-shaped parts 102 and 104 support two lateral earpieces 101 and 105. These earpieces 101 and 105 are positioned close to the user's ears when the device is in place on the user's head. The earpieces contain the electronic circuits that convert the analogue signals from the 108 electrodes into digital signals and also support preformed metal lamellae 107. These preformed metal lamellae 107 branch out from the earpieces 101 and 105 and each has an electrode 108 at its free end for the acquisition of the EEG signal in the position of interest. The combination of flexible arc-shaped parts 102, 104 and metal lamellae 107 makes the device easily and conveniently adaptable to the different possible head shapes of the users. In addition, the elasticity of the lamellae is advantageous for holding the electrodes in position and for exerting an adequate contact force. Once the lamellae are deformed with respect to their rest position, they apply a force to the electrodes towards the user's head. In order to further improve contact with the user's scalp and comfort during use, the invention proposes a solution to add further degrees of freedom to the contact end of electrode 108 and, in this way, adapt the orientation of electrode 108 to the generally uneven surface of the user's head. Figures 2, 3 and 4 illustrate the proposed inventive electrode. The electrode has an electrode body 200 consisting of two covers 201, 202 which are used to house the electronic circuit board 209. The electronic circuit board 209 inserted in the electrode is used to amplify the signals taken from the user and decouple the impedance with the rest of the circuit. A first amplification stage is necessary inside the electrode itself in the case of dry electrode to ensure good EEG signal quality at the level of the analogue/digital converter.

The electrode has a contact element 203. This contact element has several tips, 207, which are used to make contact with the scalp through the user's hair. The back of the 203 contact element holds a 206 compression spring in place. Contact element 203 can be fixed to the electrode housing by means of a screw 204. Screw 204 is preferably metallic and in this case has dimensions M1.4 x 10 mm. Screw 204 is not used to provide an electrical connection between the contact element 203 and the electronic circuit board 209, instead it is used only to prevent the contact element 203 from moving away due to the thrust exerted by the compression spring 206. A nut cage inside the cover 202 allows the nut 211 to be housed. Nut 211 is used to secure screw 204 which passes through a hole in the contact element 203. Since the hole in contact element 203 has a conical shape, contact element 203 is movable relative to screw 204.

The spring 206 shown here is a conical spring, but any other technically equivalent elastic element could be used. The spring 206, which in this case has a length of 10 mm at rest, has a double function, namely, on the one hand it serves to fix the rest position of the contact element with respect to the electrode body, and on the other hand it serves to ensure the electrical connection between the contact element itself and the electronic circuit board.

The electronic circuit board has a through-hole traversed by the screw 204. This through-hole delimits a circular crown area made of a conductive material on the surface of the circuit board. This conductive surface provides an electrical connection with the electronic circuit board. The spring 206 contacts the conductive surface of the circuit board 209 on the one hand and the contact element 203 on the other hand. The use of a conical spring with different diameters at both ends allows the spring to adapt to the average diameter of the conductive surface of the electronic circuit board and to the seat of the contact element.

When using the device, the force exerted by the preformed lamella 107 causes the spring 206 to compress slightly. This compression moves the contact element 203 away from the screw head 204, allowing small rotations of the contact element with respect to the transverse axes of the electrode. Indeed, this structure provides two additional degrees of rotational freedom to contact element 203. These degrees of freedom provide better adhesion when the user's cranial surface is not perpendicular to the longitudinal axis of the electrode.

Figure 5 shows a view of the electrode in Figure 2a and its preformed lamella designed to press the electrode onto a user's head surface. An additional nut 212 caged in the lid 202 and a screw 205 threaded through a hole in the lamella can be used to connect the lamella 107 to electrode 200. The screw 205 is also responsible for assembling the two covers 201 and 202 together with screw 210. To connect the electronic board 209 to the acquisition circuit, an electrical cable 214 can be used which runs parallel to the lamella 107. This cable 214 enters the electrode body via a through-hole 208 in the cover 202.

## Claims

1. Dry electrode (108) for measuring bioelectrical signals, comprising an electrode body (200) and a contact element (203) intended to be placed into contact with a portion of the scalp of a user, wherein said contact element (203) is connected in an articulated and sliding manner with respect to said electrode body (200) and is urged by elastic means (206) interposed between said electrode body (200) and said contact element (203), for adjusting the inclination of said contact element (203) with respect to a portion of a user's scalp, said electrode body (200) includes an electronic circuit (209) for amplifying the electrical signal detected by said contact element (203) of the electrode (108) and for decoupling the impedance, said elastic means (206) interposed between said electrode body (200) and said contact element (203) consist of, or include, an electrically conductive material, wherein the electrical signal detected by said contact element (203) is transmitted to the electronic circuit (209) by said elastic means (206), **characterized in that** for the articulated connection of said contact element (203) to said electrode body (200) there are provided a conical through-hole in the contact element (203) and an elongated element (204) inserted in said conical through-hole in the contact element, wherein said elongated element (204) is fixed at one end to said electrode body (200) and has at its opposite end an enlargement which engages with said conical hole of the contact element (203) in such a way to allow said contact element (203) to rotate and slide relative to the axis of the elongated element.

2. Dry electrode according to claim 1, **characterized in that** said elastic means (206) are formed by a spring of electrically conductive material which is placed between said contact element (203) and an electrically conductive part of said electronic circuit (209) in said electrode body (200).

3. Dry electrode according to claim 2, **characterized in that** said spring (206) is a conical helical spring and the larger diameter end of said spring (206) is in contact with said contact element (203), while the smaller diameter end is in contact with an electrically conductive part of said electronic circuit (2009) in said electrode body (200).

4. Dry electrode according to claim 1, **characterized in that** said elongated element (204) consists of a screw with a tapered head, whose edge is in contact with the wall of the conical through-hole of said contact element (203), and a stem which faces the electrode body (200) and has a threaded part that engages a threaded hole in the electrode body (200).

5. Device (100) for reading bioelectric signals through a plurality of electrodes (108) according to claim 1, said device consisting of a central body (103) containing an electronic board, from which two flexible parts (102, 104) extend to form an arc intended to be placed on the head of a user, wherein said flexible parts (102, 104) are designed to support two lateral earpieces (101, 105) positioned near the ears of the user when the device is used, said earpieces (101, 105) containing two electronic circuit boards suitable for acquiring analogue bioelectric signals and converting them into digital signals, and said lateral earpieces (101, 105) supporting preformed lamellae (107) each of which carries at its free end an electrode (108) for the acquisition of the bioelectric signal.

6. Device according to claim 5, **characterized in that** each preformed lamella (107) is configured so as to determine at least a partial compression of the elastic element (206) between said contact element (203) and said electrode body (200) when the device is applied to the head of a user, wherein said at least partial compression of said elastic element (206) determines an increase in the degrees of freedom of said contact element (203) with respect to the body of the electrode (200) and allows said contact element (203) to adjust to the user's head surface.
